# EUROPEAN PATENT APPLICATION

(11) **EP 2 596 704 A1**
(43) Date of publication of application: **29.05.2013**
(21) Application number: 11190863.8
(22) Date of filing: 28.11.2011
(51) Int. Cl.: A23L 1/30, A23K 1/16, A23K 1/18, A23L 1/03, A61K 31/33, A61P 37/04, A61P 39/06

(54) **Increasing the bioavailability of hydroxycinnamic acids**

(71) Applicant: Nestec S.A., 1800 Vevey (CH)
(72) Inventor: Renouf, Mathieu, 1814 La Tour-de-Peilz (CH); Williamson, Gary, HARROGATE, Yorkshire HG2 7AX (GB); Dionisi, Fabiola, 1066 EPALINGES (CH); Poquet, Laure, 1077 Servion (CH)
(74) Representative: Marquardt, Ulf

(57) **Abstract**

The present invention generally relates to the field of nutrition, health and wellness. For example, the present invention relates to hydroxycinnamic acids and their health benefits. The present invention discloses compositions that allow increasing the bioavailability and/or bioefficacy of hydroxycinnamic acids. According to the invention, this can be achieved by co-administering at least one flavonoid and/or its glycoside conjugate with hydroxycinnamic acids.

## Description

The present invention generally relates to the field of nutrition, health and wellness. For example, the present invention relates to hydroxycinnamic acids and their health benefits. The present invention discloses compositions that allow increasing the bioavailability and/or bioefficacy of hydroxycinnamic acids. According to the invention, this can be achieved by co-administering at least one flavonoid and/or its conjugate with hydroxycinnamic acids.

Hydroxycinnamic acids are highly abundant phenolic compounds in our diet, and they are ubiquitously found in fruits, vegetables and coffee. Estimates of daily intake of hydroxycinnamic acids could be very high (500-1000 mg/d), especially among coffee drinkers. In vitro and in vivo studies have suggested that the consumption of hydroxycinnamic acids is associated with beneficial effects linked to their antioxidant capacity [Natella F, et al., J Agric Food Chem 2002;50:6211-6; Natella F, et al., Am J Clin Nutr 2007;86:604-9; Poquet L, et al., Arch Biochem Biophys 2008;476:196-204.].

However, in humans, the first-pass metabolism of hydroxycinnamic acids plays a major role in limiting their bioavailability. Phase II enzymes in particular, are directly involved in the inactivation of dietary hydroxycinnamic acids by forming conjugates of sulfate, glucuronide or amino acid [Poquet L, et al., Biochem Pharmacol 2008;75:1218-29]. Several human and in vitro mechanistic studies showed that absorbed hydroxycinnamates are extensively metabolized in the intestine and liver, and identified sulfotransferases (SULTs) as the major metabolic enzymes involved. As a consequence, sulfate conjugates of hydroxycinnamic acids are the major forms detected in human plasma and urine, while the free acids are found in low levels. In humans, cytosolic SULTs consist of 11 members that catalyze the sulfation of low molecular weight endogenous compounds and xenobiotics [Blanchard RL et al., Pharmacogenetics 2004;14:199-211]. Hydroxycinnamic acid sulfates are the major products formed in the human liver and intestinal homogenates, indicating that both organs contributed to hydroxycinnamic acid sulfation in humans. Sulfation is targeted at the phenolic hydroxyl groups, which are a major determinant of the strong antioxidant capacity of hydroxycinnamic acids [Giacomelli C, et al., Redox Rep 2004;9:263-9]. Hence, sulfation of hydroxycinnamic acids has a significant impact on their bioavailability and reducing their bioefficacy.

As, however, free hydroxycinnamic acids exhibit the beneficial antioxidant capacity, it would be desirable to have available a composition that allows to increase the bioavailability of hydroxycinnamic acids.

The present inventors have addressed this need.

It was consequently the objective of the present invention to improve the state of the art and to provide a natural composition that allows increasing the bioavailability of hydroxycinnamic acids and that consequently exhibits an improved antioxidant capacity.

The present inventors were surprised to see that they could achieve this objective by the subject matter of the independent claims. The dependant claims further develop the idea of the present invention.

The inventors have demonstrated the inhibitory effects of flavonoids and their conjugates on the sulfation of five major dietary hydroxycinnamic acids (caffeic, dihydrocaffeic, dihydroferulic, ferulic and isoferulic acids).

Inhibitory effects of eleven dietary flavonoids aglycones on sulfation of hydroxycinnamic acids were shown in human intestine and liver homogenates. The effect of quercetin-3-glucuronide, quercetin-7-glucuronide and quercetin-3'-sulfate, the major quercetin conjugates in human plasma, on sulfation was shown in human liver homogenates.

The inhibitory effect of luteolin, quercetin and quercetin conjugates on hydroxycinnamic acids sulfation was also demonstrated in the human hepatoma cell line, HepG2, as a model for human liver.

Based on these findings, the inventors believe that the inhibition of SULTs is a possible strategy to improve the bioavailability of unconjugated hydroxycinnamic acids, which in turn, leads to an enhanced bioefficacy. In particular, human gut SULTs may be inhibited by dietary doses of flavonoids, since the local concentrations of flavonoids in gut is much higher than that in plasma [Manach C, et al., Am J Clin Nutr 2005;81:230S-42S].

Human liver is another important site of hydroxycinnamic acid sulfation. However, in vivo, the flavonoids are extensively metabolized into conjugates.

The inventors have now found that flavonoid conjugates are also effective inhibitors of sulfation of hydroxycinnamic acids after intestinal absorption.

With flavonoids having the potential to modulate the bioavailability of hydroxycinnamic acids via the inhibition of SULT1A in the human intestine and liver, the co-consumption of hydroxycinnamic acids together with flavonoids allows an increase of the concentration of unconjugated hydroxycinnamic acids in the circulation, and as a consequence an increase of the bioefficacy following the ingestion of hydroxycinnamic acids.

Consequently, the present invention relates in part to a composition comprising at least one dietary hydroxycinnamic acid for use in the treatment, alleviation or prevention of disorders linked to oxidative stress, inflammatory processes and/or a reduced immune response, wherein at least one flavonoid and/or its conjugate is added to the hydroxycinnamic acid containing composition.

The present invention also relates to the use of at least one dietary hydroxycinnamic acid in combination with at least one flavonoid and/or its conjugate for the preparation of a composition to treat, alleviate or prevent disorders linked to oxidative stress, inflammatory processes and/or a reduced immune response.

The present invention also relates to the use of at least one flavonoid and/or its glycoside conjugate for the preparation of a composition to treat, alleviate or prevent disorders linked to oxidative stress, inflammatory processes and/or a reduced immune response. The at least one flavonoid will then increase the bioavailability and bioefficacy of the hydroxycinnamic acids that are consumed with the daily nutrition.

Hence, the present invention also relates in part to a composition comprising at least one flavonoid and/or its glycoside conjugate for use in the treatment, alleviation or prevention of disorders linked to oxidative stress, inflammatory processes and/or a reduced immune response. This composition may be to be administered before, after or during the consumption of foods or drinks that naturally contain hydroxycinnamic acids.

The inventors have found that the at least one flavonoid and/or their conjugates inhibits at least partially the sulfation of dietary hydroxycinnamic acids.

The flavonoid may be selected from the group consisting of apigenin, luteolin, kaempferol, isorhamnetin, quercetin, hesperetin, genistein, daidzein, (+)-catechin, (-)-epicatechin, phloretin, or combinations thereof.

The inventors found genistein, daidzein, apigenin, phloretin, luteolin and/or quercetin were particularly effective under the conditions tested.

Importantly, the inventors found that not only flavonoids but also their glycoside conjugates can be used for the purpose of the present invention.

As conjugates, glycoside conjugates may be used.

As glycosides, rhamnose, glucose, galactose, xylose, arabinose, fucose or combinations of theses glycosides, for example rutinoside may be used.

Examples of glycoside conjugates of flavonoids may be rutin (quercetin-3-0-rutinoside), and/or daidzin (daidzein-7-0-glucoside).

In therapeutic applications, compositions are administered in an amount sufficient to at least partially cure or arrest the symptoms of the disease and its complications. An amount adequate to accomplish this is defined as "a therapeutically effective dose". Amounts effective for this purpose will depend on a number of factors known to those of skill in the art such as the severity of the disease and the weight and general state of the patient.

In prophylactic applications, compositions according to the invention are administered to a patient susceptible to or otherwise at risk of a particular disease in an amount that is sufficient to at least partially reduce the risk of developing a disease. Such an amount is defined to be "a prophylactic effective dose". Again, the precise amounts depend on a number of patient specific factors such as the patient's state of health and weight.

In the present applications the hydroxycinnamic acid and/or the flavonoid and/or their glycoside conjugates are administered in a therapeutically or prophylactic effective dose. Such dosages can be accurately determined by skilled artesians.

For example, the composition of the present invention may comprise at least 1mg hydroxycinnamic acid, at least 10mg hydroxycinnamic acid, or at least 50mg hydroxycinnamic acid per serving.

For example, the composition of the present invention may comprise at least 10 mg, at least 100 mg, or at least 1000mg flavonoids and/or their conjugates per serving.

For example, flavonoids and/or their conjugates and hydroxycinnamic acids may be present in the composition in a molar ratio in the range of 100:1 to about 1:1, or 100:1 to about 10:1. While more flavonoids will protect hydroxycinnamic acids from inactivation through the formation of sulfate conjugates in a dose dependant manner, it was found that the above ratios are usually ideal for most applications.

The at least one flavonoid and/or their conjugates may be provided in any form, e.g., as chemically synthesized compounds or as compounds purified from natural sources.

It is however preferred that the at least one flavonoid and/or their conjugates are added to the composition in the form of a natural food product or an extract thereof.

This will underline the naturality of the composition.

Natural sources of the at least one flavonoid and/or their conjugates may be selected from the group consisting of black or green tea, capers, lovage, apples, onion, in particular red onion, red grapes, citrus fruit, tomato, broccoli, raspberry, bog whortleberry, lingonberry, cranberry, chokeberry, sweet rowan, rowanberry, sea buckthorn berry, crowberry, prickly pear cactus fruit, or combinations thereof.

Similarly, also the hydroxycinnamic acid may be provided in any form, e.g., as chemically synthesized compounds or as compounds purified from natural sources. Also here it is preferred if the hydroxycinnamic acid is provided as natural food product or extract thereof.

For example, hydroxycinnamic acids may be obtained from or provided as coffee, cocoa, vegetables or fruits.

The hydroxycinnamic acid is preferably a dietary hydroxycinnamic acid and may be selected from the group consisting of caffeic acid, dihydrocaffeic acid, dihydroferulic acid, ferulic acid, isoferulic acid, or combinations thereof.

Preferred examples of dietary hydroxycinnamic acids are caffeic acid, ferulic acid, or combinations thereof.

The composition of the present invention may be for use in the treatment, prevention or alleviation of disorders linked to oxidative stress, inflammatory processes and/or a reduced immune response.

These disorders may be selected from the group consisting of hyperglycemia, type 2 diabetes mellitus, cardiovascular disorders, acute immune and/or inflammatory responses, skin cells damage caused by ultraviolet (UV) radiation, accelerated cell aging, and combinations thereof.

The composition of the present invention may also be used for cosmetic purposes, e.g., as a cosmetic composition that is ingested.

As such the present invention also relates to a cosmetic use of a composition comprising at least one dietary hydroxycinnamic acid and at least one flavonoid and/or its conjugate to treat or prevent oxidative damage, wherein the composition is to be administered orally.

The composition may be any composition suitable for human or animal use. For example, the composition may be selected from the group consisting of food products, drinks, petfood products, nutraceuticals, food additives or cosmetic products.

As such, the composition may be to be administered to humans or animals, for example pet animals such as cats or dogs.

The composition may be consumed at any time of the day. It may be preferred, however, to administer the compositions of the present invention in the morning to prepare the body for the challenges of the day.

To ensure a good protection of the body throughout the days, it may also be preferred to consume the composition of the present invention also during the day, for example with the meals. As such the compositions of the present invention may be to be administered in the morning, at lunchtime and in the evening.

Those skilled in the art will understand that they can freely combine all features of the present invention described herein, without departing from the scope of the invention as disclosed. In particular, features described for the uses of the present invention may be applied to the composition of the present invention and vice versa.

Further advantages and features of the present invention are apparent from the following Examples and Figures.

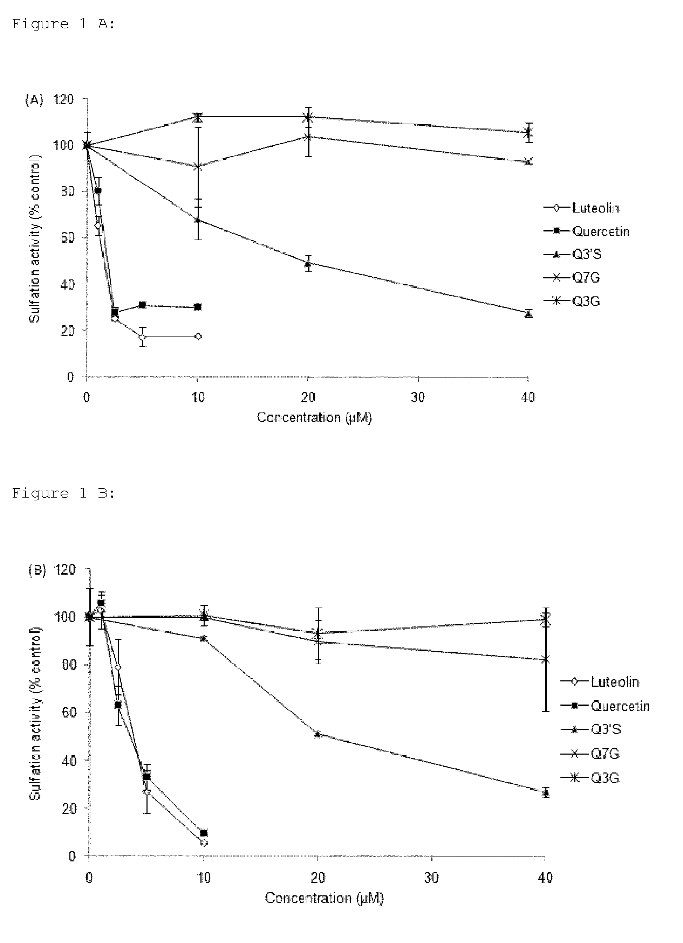

Figure 1 A and B shows the inhibitory effect of luteolin, quercetin, and quercetin conjugates on sulfation of caffeic acid (A) and ferulic acid (B) in HepG2 cells. Caffeic and ferulic acids (10 µM) were incubated in the presence of luteolin, quercetin or quercetin conjugates for 4 h.

### Examples:

### 1.1. Inhibition of hydroxycinnamic acids sulfation by flavonoids in human liver and intestinal S9

The incubation mixture, in a final volume of 50 µL, consisted of 100 mM potassium phosphate buffer (pH 7.4), with 100 µM vitamin C, 100 µM PAPS and 1 mM DTT. Human liver S9 and intestinal S9 homogenates were used at 1 mg/mL and 0.4 mg/mL, respectively. The flavonoids were added from a 5 mM stock solution dissolved in DMSO, with final DMSO concentration equalized to 0.2 %. Quercetin-3-glucuronide, quercetin-7-glucuronide and quercetin-3'-sulfate were dissolved in water. After a 15 min pre-incubation period, the reaction was initiated by adding 10 µM cinnamic acids and 25 µM dihydrocinnamic acids from a 50 mM stock solution in DMSO. To inhibit hydrolysis of quercetin glucuronides, in some analyses, 5 mM saccharolactone was added. After 30 min incubation in a 37 °C water bath, the reaction was stopped by addition of 10 µL ice-cold acetonitrile containing 500 mM HCl. Controls were treated under identical condition and consisted of samples with 0.2 % DMSO (final concentration) added to the buffer. Samples were stored at -70 °C until analysis.

### 1.2. HepG2 Cell culture

HepG2 cells (ATCC) were routinely cultured in 75 cm² cell culture flasks at 37 °C under a humidified 5 % CO2 / 02 atmosphere. The culture media consisted of Eagle's Minimum Essential Medium (EMEM) media supplemented with 10% fetal bovine serum (Sigma-Aldrich) and 100U/ml penicillin-streptomycin. All experiments were performed with HepG2 cells between passages 80 to 95. For metabolic studies, HepG2 cells were seeded into 12-well plates at a cell density of 2 x 10⁵ per well. The cell monolayers were allowed to grow over 96 h before they were used for experiments. Hydroxycinnamic acid metabolism experiments were carried out in serum-free media with 100 µM vitamin C and 1.8 mM CaC12, adjusted to pH 6.5. Cinnamic acids (10 µM) and dihydrocinnamic acids (25 µM) were added from a 50 mM stock solution in DMSO. Quercetin and luteolin (5 mM) were also dissolved in DMSO and added to the media to give a final DMSO concentration of 0.25 %. 0.4 mL of hydroxycinnamic acids, with or without the inhibitors, were added to the HepG2 cells and incubated for 4 h at 37 °C. The incubation media were then collected, acidified with 1 mM vitamin C and dried under vacuum. The residue was extracted by sonication for 5 min and vortex for 1 min, first with 500 µL acetonitrile, followed by 500 µL methanol. The extracts were combined and centrifuged at 17,000 g for 10 min. The supernatant was evaporated under vacuum. Prior to HPLC analysis, the dried residue was re-dissolved in 100 µL of initial mobile phase.

### 1.3. HPLC methodology for hydroxycinnamic acids

HPLC analyses were carried out using the Agilent 1200 series liquid chromatography system. For the analysis of caffeic acid, ferulic acid, isoferulic acid, dihydroferulic acid and their conjugates, chromatography was performed with a Zorbax XDB-C18 column (4.6 x 150 mm, 5 µm). The mobile phase consisted of 20 mM ammonium formate, pH 2.8 (A) and methanol (B). For the analysis of caffeic acid and conjugates, samples were eluted at 1 mL/min with 5 % to 25 % B in 20 min, followed by 80 % B in 2 min and back to 5 % B for 3 min. For the analysis of ferulic and isoferulic acid and their conjugates, the gradient was from 10 % to 20 % B in 10 min, to 60% B in 15 min, then set at 80 % B for 2 min and back to 10 % B for 3 min, at 1 mL/min. Dihydroferulic acid and conjugates analysis was carried out at 1 mL/min, from 10 % to 20 % B in 15 min, to 60 % B in 10 min, up to 80 % B for 2 min and finally to 10 % B for 3 min. For dihydrocaffeic acid and conjugates, the analyses were performed with a Zorbax XDB-C18 column (4.6 x 50 mm, 1.8 µm) with 20 mM ammonium formate, pH 4.5 (A) and methanol (B) as the mobile phase. The gradient started at 3 % (B) kept for 15 min, followed by an increase to 40 % B in 5 min, and then returned to 3 % B for 5 min. Samples were centrifuged and 25 µL of the supernatant were injected into the column. UV-detection carried out at 280 nm and 310 nm using photodiode array detector. Caffeic, ferulic and isoferulic acid were quantified at 310 nm, dihydroferulic acid and dihydrocaffeic acid at 280 nm.

### 2. Results

The inhibitory effect (IC₅₀) of flavonoids and their conjugates is shown in Table 1, 2 and 3.

**Table 1. Inhibition of caffeic acid and ferulic acid sulfation by flavonoids in human intestinal S9**

| Flavonoids | IC₅₀ (µM) for the inhibition of sulfation of | |
|---|---|---|
| | Caffeic acid | Ferulic acid |
| Apigenin | 0.96 | 2.3 |
| Luteolin | 1.3 | 3.0 |
| Kaempferol | 2.5 | 3.8 |
| Isorhamnetin | 4.4 | 4.7 |
| Quercetin | 4.2 | 5.2 |
| Hesperetin | 3.5 | 5.1 |
| Genistein | 0.77 | 1.5 |
| Daidzein | 0.72 | 2.3 |
| (+)-Catechin | 5.8 | 7.0 |
| (-)-Epicatechin | 7.6 | 11 |
| Phloretin | 0.84 | 2.3 |

**Table 2. Inhibition of caffeic acid and ferulic acid sulfation by flavonoids in human liver S9**

| Flavonoids | IC₅₀ (µM) for the inhibition of sulfation of | |
|---|---|---|
| | Caffeic acid | Ferulic acid |
| Apigenin | 0.46 | 0.88 |
| Luteolin | 0.08 | 0.53 |
| Kaempferol | 0.92 | 3.9 |
| Isorhamnetin | 2.0 | 3.7 |
| Quercetin | 0.41 | 0.64 |
| Hesperetin | 4.3 | 5.3 |
| Genistein | 0.59 | 1.0 |
| Daidzein | 0.65 | 0.62 |
| (+)-Catechin | 4.2 | 4.4 |
| (-)-Epicatechin | 7.4 | 7.3 |
| Phloretin | 0.99 | 1.0 |

**Table 3. Inhibition of caffeic acid and ferulic acid sulfation by quercetin conjugates in liver S9**

| Conjugate | IC₅₀ (µM) for the inhibition of sulfation of | |
|---|---|---|
| | Caffeic acid | Ferulic acid |
| Quercetin-3-glucuronide | 11 | 12 |
| Quercetin-7-glucuronide | 9.6 | 8.0 |
| Quercetin-3'-sulfate | 6.6 | 6.7 |

Flavonoids were found to be potent inhibitors of sulfation of hydroxycinnamic acid. Isoflavones were the strongest inhibitors in intestinal S9, while quercetin and luteolin were the most effective inhibitors in liver S9. Quercetin conjugates, as the forms found in blood, were also effective, with IC50 values in the low micro-molar range.

The effect of flavonoids and conjugates on the sulfation of hydroxycinnamic acids in HepG2 cells was summarized in Figure 1A and 1B.

## Claims

1. Composition comprising at least one dietary hydroxycinnamic acid for use in the treatment, alleviation or prevention of disorders linked to oxidative stress, inflammatory processes and/or a reduced immune response, wherein at least one flavonoid and/or its glycoside conjugate is added to the hydroxycinnamic acid containing composition.

2. Composition in accordance with claim 1, wherein the at least one flavonoid and/or their conjugates inhibits at least partially the sulfation of dietary hydroxycinnamic acids.

3. Composition in accordance with one of the preceding claims comprising at least 1mg hydroxycinnamic acid per serving.

4. Composition in accordance with one of the preceding claims comprising flavonoids and hydroxycinnamic acid in a molar ratio in the range of 100:1 to about 10:1.

5. Composition in accordance with one of the preceding claims, wherein the at least one flavonoid and/or their conjugates are added to the composition in the form of a natural food product or an extract thereof.

6. Composition in accordance with claim 5, wherein the natural food is selected from the group consisting of black or green tea, capers, lovage, apples, onion, in particular red onion, red grapes, citrus fruit, tomato, broccoli, raspberry, bog whortleberry, lingonberry, cranberry, chokeberry, sweet rowan, rowanberry, sea buckthorn berry, crowberry, prickly pear cactus fruit, or combinations thereof.

7. Composition in accordance with one of the preceding claims, wherein the dietary hydroxycinnamic acid is obtained from coffee, cocoa, vegetables or fruits.

8. Composition in accordance with one of the preceding claims, wherein the dietary hydroxycinnamic acid is selected from the group of caffeic acid, dihydrocaffeic acid, dihydroferulic acid, ferulic acid, isoferulic acid, or combinations thereof.

9. Composition in accordance with one of the preceding claims, wherein the flavonoid is selected from the group consisting of quercetin and its glycoside conjugates.

10. Composition in accordance with one of the preceding claims, wherein disorders linked to oxidative stress, inflammatory processes and/or a reduced immune response are selected from the group consisting of hyperglycemia, type 2 diabetes mellitus, cardiovascular disorders, acute immune and/or inflammatory responses, skin cells damage caused by ultraviolet (UV) radiation, accelerated cell aging, and combinations thereof.

11. Composition in accordance with one of the proceeding claims, wherein the composition is selected from the group consisting of food products, drinks, petfood products, nutraceuticals, food additives or cosmetic products.

12. Composition in accordance with one of the proceeding claims to be administered in the morning.

13. Cosmetic use of a composition comprising at least one dietary hydroxycinnamic acid and at least one flavonoid and/or its conjugate to treat or prevent oxidative damage, wherein the composition is to be administered orally.
